# EUROPÄISCHE PATENTSCHRIFT

(11) **EP 0 694 578 B1**
(45) Veröffentlichungstag und Bekanntmachung des Hinweises auf die Patenterteilung: **29.09.1999**
(21) Anmeldenummer: 95109598.3
(22) Anmeldetag: 21.06.1995
(51) Int. Cl.: C08L 63/00, C08G 59/40, C09D 5/44, C08J 3/03, C08G 59/24, C08G 18/64

(54) **Wässrige Kunstharzdispersion**
Aqueous synthetic resin dispersion
Dispersion aqueuse de résine synthétique

(30) Priorität: 30.06.1994 DE 4422869; 10.10.1994 DE 4436093
(43) Veröffentlichungstag der Anmeldung: 31.01.1996
(73) Patentinhaber: HOECHST AKTIENGESELLSCHAFT, 65926 Frankfurt am Main (DE)
(72) Erfinder: Neumann, Uwe, Dr., D-65307 Bad Schwalbach (DE); Hönel, Michael, Dr., D-65189 Wiesbaden (DE); Völker, Achim, Dr., D-65197 Wiesbaden (DE); Walz, Gerd, Dr., D-65207 Wiesbaden (DE); Wehner, Susanne, D-65606 Villmar (DE); Ziegler, Peter, Dr., D-55130 Mainz (DE)
(74) Vertreter: Zounek, Nikolai, Dipl.-Ing.

(56) Entgegenhaltungen:
- EP-A- 0 189 728
- EP-A- 0 622 389

## Beschreibung

Bei der Elektrotauchlackierung werden vorzugsweise solche Elektrotauchlacke eingesetzt, die als Bindemittel kationische, durch Amine modifizierte Epoxidharze als wasserlösliche Basisharzkomponente und mit Alkoholen und/oder Aminen blockierte Polyisocyanate als Vernetzer enthalten.

Aus Korrosionsschutzgründen werden heutzutage bevorzugt auf Basis von Bisphenol A aufgebaute Epoxidharze eingesetzt, die mit Aminen in protischen Lösemitteln wie Alkoholen und Glykolen und/oder aprotischen Lösemitteln wie Ketonen und Aromaten umgesetzt werden, um sie wasserlöslich zu machen.

Gerade die auf Basis von Bisphenol A aufgebauten Epoxidharze eignen sich hervorragend zum Korrosionsschutz und werden daher bevorzugt zur Synthese von aminmodifizierten Epoxidharzen eingesetzt.

Zur Herstellung der einzelnen Bindemittelkomponenten müssen aus Viskositätsoder Reaktivitätsgründen Lösemittel eingesetzt werden. So werden bei der Synthese der Basisharze protische (z.B. Alkohole, Glykole) und aprotische Lösemittel (z.B. Ketone, Ester oder Aromaten) und bei der Synthese der Vernetzerkomponente aprotische Lösemittel verwendet.

Bekannterweise wird aber mit steigendem Lösemittelgehalt in kathodischen Tauchlackier-("KTL")-Bädern der Umgriff (Hohlraumbeschichtung) verschlechtert, es treten Überbeschichtungen auf und Lösemittel gelangen vermehrt in das Abwasser und/oder die Abluft. Daher müssen die Lösemittel den Bindemitteln vor und/oder nach dem Dispergieren in Wasser z.B. durch Destillation oder Ultrafiltration entzogen werden. Dabei verbleibt verfahrensbedingt ein Teil der Lösemittel in den Dispersionen und es fallen, besonders beim sogenannten "Strippen" aus wäßriger Phase und beim Ultrafiltrieren, größere Mengen eines Lösemittel-Wasser-Gemisches an, das entsorgt werden muß.

Es bestand daher die Aufgabe, ein vereinfachtes und ökologisch unbedenkliches Verfahren zur Herstellung wäßriger KTL-Bindemittel-Dispersionen zu entwickeln, dieweitgehend frei von organischen Lösemitteln sind.

In der Patentanmeldung DE-A 43 14 297 wurden verbesserte Kunstharzdispersionen gefunden, die Lackierungen mit einer guten Beständigkeit beispielsweise im Salzsprühtest ergeben.

Optimierungsbedürftig sind bei den in DE-A 43 14 297 genannten und als Lösemittel zur Bindemittelsynthese verwendeten, ungesättigten Monomeren deren Verträglichkeit bzw. "Wechselwirkung" mit den polaren Epoxyaminaddukten auf Basis von Epoxidharzen.

Diese Unverträglichkeit drückt sich beispielsweise aus in der erhöhten Viskosität a) der in Monomeren (speziell Vinylaromaten wie Styrol) hergestellten Epoxyaminaddukte und b) in der Schmelzenviskosität während des Einbrennvorgangs aus.

Überraschenderweise konnte die Wechselwirkung zwischen Epoxyaminaddukt und Monomer (speziell Vinylaromaten wie Styrol) dadurch optimiert werden, daß man entsprechend einer Friedel-Crafts-Alkylierung Bisphenol A mit Alkenylaromaten umsetzt und die derart seitenkettenmodifizierten Bisphenol A-Derivate a) zum Aufbau eines Epoxidharzes bzw. b) zum Aufbau eines Epoxyaminaddukts verwendet, wobei die Herstellung dieser Bindemittelkomponente vorzugsweise in Gegenwart ungesättigter Monomere erfolgt, die anschließend durch Emulsions- bzw. Suspensionspolymerisation in wasserunlösliche Polymere überführt werden. Man erhält auf diese Weise wäßrige Dispersionen, die überraschenderweise im pH-Bereich von 5 bis 8 stabil sind.

Gegenstand der Erfindung sind wäßrige Kunstharzdispersionen enthaltend A) ein ionisches Harz auf Basis von nach Friedel-Crafts aralkylierten Epoxidharzen, B) ein verkapptes Polyisocyanat und ein Polymer aus mindestens einem radikalisch polymerisierbaren olefinisch ungesättigten Monomeren C).

Die erfindungsgemäßen wäßrigen Kunstharzdispersionen werden hergestellt, indem man eine Mischung von mindestens einem Monomer C), einem ionischen Epoxidharz A) und einem verkappten Polyisocyanat B) in eine wäßrige Dispersion überführt und radikalisch polymerisiert.

Dabei sind ein Teil oder alle der zur Synthese des Epoxidharzes eingesetzten Bisphenole mit je mindestens einer Aralkylgruppe substituiert.

Als ionisches Harz A) kommen sowohl anionische als auch kationische Harze in Frage, wobei kationische Harze aufgrund des besseren Umgriffs und Korrosionsschutzes bevorzugt sind. Die kationischen Harze enthalten bevorzugt Gruppen mit einem aktiven Wasserstoffatom wie Hydroxyl-, primäre oder sekundäre Amino- und Thiol-Gruppen. Diese Gruppen dienen als reaktive Zentren bei der Härtung des Lacks mit Vernetzungsmitteln wie beispielsweise blockierten Polyisocyanaten.

Bei der Komponente A) handelt es sich bevorzugt um ein durch Neutralisation mit organischen Säuren wasserverdünnbares Amino-Epoxidharz. Solche Amino-Epoxidharze weisen im allgemeinen eine Aminzahl von 30 bis 150 mg KOH/g, eine Hydroxylzahl von 50 bis 500 mg KOH/g und eine zahlenmittlere Molmasse (Mn) von 250 bis 10000, bevorzugt 300 bis 5000 g/mol auf. Die untere Grenze der Aminzahl sollte bevorzugt 45, besonders bevorzugt 70, die obere Grenze dagegen sollte bevorzugt bei 120, besonders bevorzugt bei 100 mg KOH/g liegen. Ist die Aminzahl zu niedrig, so ist die Löslichkeit zu gering oder es entstehen durch einen zu hohen Neutralisationsgrad zu saure pH-Werte in den Abscheidungsbädern. Ist die Aminzahl zu hoch, so bildet sich bei der Abscheidung ein schlecht haftender Film oder eine blasige Oberfläche.

Beispiele für Amino-Epoxidharze sind Umsetzungsprodukte von Epoxidgruppenhaltigen Harzen mit vorzugsweise endständigen Epoxidgruppen aus den Gruppen Polyglycidyläther, Polyglycidylester und Polyglycidylamine mit gesättigten und/oder ungesättigten sekundären und/oder primären Aminen bzw. Aminoalkoholen. Diese können durch mindestens eine primäre und/oder sekundäre Hydroxylgruppe, durch die Dialkylaminogruppe und/oder durch eine primäre Aminogruppe, die durch Ketiminbildung vorübergehend geschützt wird, modifiziert sein.

Hydroxylgruppenhaltige Amino-Epoxidharze werden zweckmäßig aus Polyglycidyläthern mit bevorzugt zwei 1,2-Epoxidgruppen pro Molekül erhalten. Unter Polyglycidyläther im Rahmen dieser Erfindung werden vorzugsweise solche Friedel-Crafts aralkylierten Polyglycidyläther der allgemeinen Formel 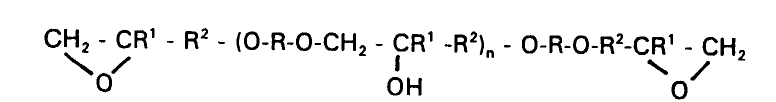 mit 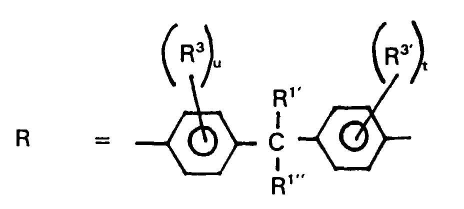 mit
- R¹,R^{1'},R^{1"} =: jeweils unabhängig voneinander -H und/oder -CₘH₂ₘ₊₁,
- R² =: -(CHR⁴)ₘ-, bevorzugt -CH₂-,
- R³,R^{3'} =: jeweils unabhängig voneinander ein Aralkylrest,
- R⁴ =: H oder ein niedriger, gegebenenfalls mit verschiedenen Substituenten versehener Alkylrest mit 1 bis 6 C-Atomen ist und
- n =: 0 bis 8, bevorzugt 1 bis 6,
- m =: 1 bis 8, bevorzugt 1,
- t,u =: jeweils unabhängig voneinander 0 bis 4,
verstanden.

Diese Polyglycidyläther werden dadurch erhalten, daß man im ersten Schritt eine oder mehrere der Verbindungen wie z.B. 4,4'-Dihydroxy-diphenylmethan (Bisphenol F), 4,4'-Dihydroxy-diphenylpropan (Bisphenol A), 4,4'-Dihydroxybenzophenon und Isomere des Dihydroxynaphthalin unter Säurekatalyse mit Alkenylaromaten im Sinne einer Friedel-Crafts-Alkylierung umsetzt und danach mit einer reaktiven Epoxidverbindung wie Halohydrinen (z.B. Epichlorhydrin und Methylepichlorhydrin) reagiert.

Die Darstellung der für die Erfindung geeigneten aralkylierten Polyphenole ist in der gleichzeitig eingereichten Anmeldung P 44 36 097.5 beschrieben.

Die derart modifizierten Bausteine für Polyglycidyläther werden dadurch erhalten, daß man a) Epihalohydrine wie Epichlorhydrin oder Methylepichlorhydrin mit aralkyliertem Diphenol umsetzt, so daß Polyglycidyläther mit einer zahlenmittleren Molmasse Mn von etwa 300 bis 5000 g/mol und einem Epoxid-Äquivalentgewicht (molare Masse dividiert durch die mittlere Anzahl der Epoxidgruppen pro Molekül) von etwa 170 bis 2500 g/mol resultieren. Polyepoxide mit geeigneter Molmasse werden entweder durch Auswahl der Mol-Verhältnisse von aralkyliertem Diphenol und z.B. Epichlorhydrin oder durch Reaktion der monomeren Diglycidylverbindungen mit weiterem aralkyliertem Bisphenol unter Zusatz von Katalysatoren wie Lewis-Säuren, Lewis-Basen oder Phosphoniumsalzen hergestellt.

Besonders bevorzugt für die Erfindung sind Epoxidharze aus Bisphenolen, die durch Umsetzung mit Styrol modifiziert worden sind.

Die Epoxidharze können vollständig oder teilweise hydriert sein, oder es können Gemische von Epoxidharzen mit unterschiedlicher Struktur und Molmasse eingesetzt werden. Weiterhin kann zum Elastifizieren ein Teil des beschriebenen Polyglycidyläthers durch aliphatische Polyglycidyläther der Formel 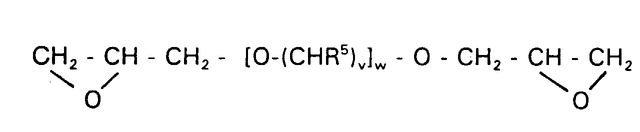 ersetzt werden, wobei
- R⁵: H oder ein niedriger, gegebenenfalls mit verschiedenen Substituenten versehener Alkylrest mit 1 bis 6 C-Atomen ist und
- v: Zahlen von 2 bis 6 und
- w: Zahlen von 5 bis 50 bedeuten.

Beispiele sind Bisglycidyläther aus Polypropylenglykol oder Polybutylenglykol mit verschiedener Molmasse. Die modifizierten Epoxidharze können auch durch Reaktion mit langkettigen Polyalkoholen wie Hexandiol-1.6, Neopentylglykol, bisäthoxyliertes Neopentylglykol, Hydroxypivalinsäure-neopentylglykolester und Bis(hydroxymethyl)-cyclohexan, Monoanhydro-pentaerythrit sowie Polytetrahydrofurandiol, Polycaprolactondiol, Polycaprolactamdiol oder Polybutadiendiol in Gegenwart von geeigneten basischen oder sauren Katalysatoren wie Borfluorid-Amin-Komplexen modifiziert werden. Während Polyalkohole mit primären OH-Gruppen sich bei geeigneter Katalyse direkt mit Polyglycidyläthern umsetzen lassen, werden sekundäre OH-Gruppen zunächst mit Di-isocyanat umgesetzt. Das erhaltene NCO-terminierte Reaktionsprodukt kann dann ohne Schwierigkeiten als Brücke zwischen 2 Mol Polyglycidyläther unter Vergrößerung des Moleküls und der Funktionalität eingebaut werden.

Das aralkylierte Epoxidharz kann zum Senken der Aminzahl auch mit gesättigten oder ungesättigten Polycarbonsäuren und/oder Hydroxyalkylcarbonsäuren modifiziert sein. Aliphatische, cycloaliphatische und/oderaromatische Polycarbonsäuren verschiedener Kettenlänge sind beispielsweise Adipinsäure, Sebacinsäure, Fumarsäure, Isophthalsäure und dimere Fettsäuren. Unter Hydroxyalkylcarbonsäuren werden Milchsäure, Dimethylolpropionsäure oder auch carboxyl- und hydroxylgruppenhaltige Polyester verstanden. Bei der Umsetzung von überschüssigem niedermolekularem Polyglycidyläther mit Polycarbonsäuren und/oder Polyalkoholen werden als Zwischenstufe modifizierte Polyglycidyläther erhalten, die dann weiter mit Aminen und/oder Aminoalkoholen reagieren.

Auch heterocyclische Polyepoxidverbindungen können verwendet werden, wie 1,3-Diglycidyl-5.5-dimethylhydantoin, Triglycidylisocyanurat oder Diepoxide aus Bisimiden. Eine andere geeignete Klasse von Polyepoxiden sind Polyglycidyläther von phenolischen Novolakharzen, wodurch die Funktionalität von 2 bis auf etwa 6 Glycidylgruppen pro Molekül gesteigert werden kann. Durch Defunktionalisieren mit langkettigen Alkylphenolen wie Dodecylphenol können zusätzlich elastifizierende Elemente eingebaut werden. Auch können Polyglycidylester von Polycarbonsäuren wie Hexahydrophthalsäurediglycidylester, Tetrahydrophthalsäurediglycidylester oder Fumarsäurediglycidylester verwendet werden.

Das Einführen der Aminogruppen erfolgt entweder durch Addition von NHreaktiven Verbindungen an die Epoxidgruppe oder durch Umsetzen der Hydroxylgruppen des Grundharzes mit basischen Monoisocyanaten, die durch Reaktion von aromatischen und/oder aliphatischen und/oder cycloaliphatischen Di- oder Polyisocyanaten mit Dialkylaminoalkanol (vgl. DE-A 27 07 405) entstehen.

Als NH-reaktive Verbindungen werden verwendet primäre Amine ausgewählt aus linearen und verzweigten Alkyl- und Alkanolaminen mit 1 bis 12 Kohlenstoffatomen im Alkyl- bzw. Alkanolrest, wie Methylamin, Äthylamin, Propylamin, Butylamin, Octylamin, Monoäthanolamin, 2-Äthylhexylamin, aus Dialkylaminoalkylaminen und Alkoxyalkylaminen mit insgesamt 3 bis 14 Kohlenstoffatomen wie Dimethylaminopropylamin, Diäthylaminoäthylamin, Dimethylaminoneopentylamin oder Methoxypropylamin und/oder bevorzugt sekundäre Amine wie Dialkylamine, Monoalkylhydroxyalkylamine, Dihydroxyalkylamine und cyclische Amine mit insgesamt 2 bis 14 Kohlenstoffatomen. Beispiele für solche Verbindungen sind Dimethylamin, Diäthylamin, Dipropylamin, Dibutylamin, Methylbutylamin, Dimethylaminopropylamin, N-Methylaminoäthanol oder Diäthanolamin oder auch cyclische Amine, wie Morpholin oder Oxazolidin. Beim Einsatz der primären Amine reagiert das Amin in Abhängigkeit von den angebotenen stöchiometrischen Verhältnissen mit 1 bis 2 Epoxidgruppen unter Molekülvergrößerung.

Weiterhin können primäre Amine der allgemeinen Formel

H₂N-CR¹R²-R³-O(CHR⁴-CHR⁵O)ₙR⁶

oder sekundäre Amine der allgemeinen Formel

H-N-CR¹R²-R³-O(CHR⁴-CHR⁵O)ₙ-R³-CR¹R²-N-H

eingesetzt werden.

In dieser Formel stehen R¹ und R² für Wasserstoff, Alkyl- oder -CH₂-OH Gruppen, R³ steht für einen linearen oder verzweigten Alkylenrest, insbesondere für einen Alkylenrest mit 1 bis 3 Kohlenstoffatomen, R⁴ und R⁵ stehen für Wasserstoff oder Alkylreste mit 1 bis 4 Kohlenstoffatomen, R⁶ steht für Wasserstoff, einen Alkyl-, Cycloalkyl- oder Phenylrest, vorzugsweise für einen Alkylrest mit 1 bis 6 Kohlenstoffatomen und n ist eine ganze Zahl von 0 bis 5. Als Beispiele für solche Monoamine werden genannt: Äthanolamin, Propanolamin, Butanolamin, Äthylenglykol(2-amino-äthyl)äther (H₂N-CH₂-CH₂-O-CH₂-CH₂-OH) und Diäthylenglykolmono(3-aminopropyl)äther (H₂N-(CH₂)₃-O-CH₂-CH₂-O-CH₂-CH₂-OH). Beim Einsatz der primären Amine reagiert das Amin mit der Epoxidgruppe in Abhängigkeit von den angebotenen stöchiometrischen Verhältnissen unter Molekülvergrößerung. Als Beispiel für Diamine werden genannt: Jeffamine® M-Serie, Jeffamine® D-Serie und Jeffamine® ED-Serie.

Weiterhin kommen Di- oder Triamine mit primären und/oder sekundären Aminogruppen in Frage, wie z.B. Laurylpropylendiamin, Talgfettpropylendiamin.

Mit sekundären Diaminen tritt eine Kettenverlängerung ein. Als sekundäre Diamine, bevorzugt langkettige Diamine, werden N,N'-Dialkyldiaminoalkane wie z.B. N,N'-Bis(isohexyl)-1,6-diaminohexan, N,N'-Bis(isohexyl)isophorondiamin,N,N'-Bis(isohexyl)-dimethylhexamethylendiamin,N,N'-Bis(isohexyl)-2-methylpentamethylendiamin, N,N'-Bis(isohexyl)-äthylendiamin und N,N'-Bis(isohexyl)-di-(4-aminocyclohexyl)methan oder Umsetzungsprodukte von gesättigten Glycidyläthern oder -estern oder Epoxyalkanen mit primären Diaminoalkanen verwendet wie das Additionsprodukt von Hexandiamin-1.6 mit 2 Mol Glycidylester der ®Versaticsäuren (in α-Stellung verzweigte Monocarbonsäuren, besonders mit 5 bis 11 Kohlenstoffatomen).

Als Monoepoxide können für diesen Zweck auch gesättigte oder ungesättigte Glycidyläther oder α-Epoxide verschiedener Kettenlänge wie Dodecan-1,2-oxid oder Butylenoxid eingesetzt werden. Die geeignete Zahl an Hydroxylgruppen entsteht dabei einmal aus der Epoxidgruppe bei der Addition der sekundären Aminogruppen, zum anderen kann sie durch Einsatz von Hydroxyalkylaminen gesteuert werden. Weiterhin können als sekundäre Diamine das Umsetzungsprodukt von 2 Mol 2-Äthylhexylamin mit 1 Mol Beckopox® EP 075 (Diglycidyläther auf Basis von Propylenoxid) und/oder 1 Mol ®Beckopox EP 140 (Epikote® 828), sowie aliphatische sekundäre Diamine auf Basis von Propylenoxid-Addukten von Diolen oder Triolen, wie z.B. Novamin®-Typen zum Einsatz kommen.

Die Molverhältnisse zwischen Epoxid- und Aminogruppen-haltigen Verbindungen sind so zu wählen, daß der vollständige Einbau des Amins gewährleistet ist, weil durchbruchartige Oberflächenstörungen beim elektrophoretischen Beschichten auftreten können, d. h. ein geringfügiger Überschuß an Epoxidgruppen ist vorteilhaft.

Alle Amine können gleichzeitig oder stufenweise mit den Epoxidgruppenhaltigen Verbindungen umgesetzt werden. Die Reaktion der Amine beginnt schon bei Raumtemperatur und ist im allgemeinen exotherm. Um eine vollständige Umsetzung zu erreichen, ist es in der Regel notwendig, die Temperatur zeitweise auf etwa 50 bis 120 °C zu erhöhen. Bei der Herstellung des Amino-Epoxid-Harzes geht man so vorteilhafterweise vor, daß man zunächst das Epoxid-Harz in einem oder mehreren radikalisch polymerisierbaren olefinisch ungesättigten Monomeren löst und dann mit den Aminen umsetzt.

Für Vernetzungsreaktionen müssen in dem Amino-Epoxid-Harz stets Hydroxylgruppen vorhanden sein. Die im Molekül vorhandene Hydroxylzahl (gemessen in mg KOH pro Gramm Festharz) ist maßgebend für die Vernetzungsfähigkeit des Films. Sie sollte über 50, vorzugsweise über 100, besonders vorteilhaft über 150 mg/g liegen. Die obere Grenze der Hydroxylzahl liegt bei 400, vorteilhafter unter 300 mg/g. Ist die Hydroxylzahl zu niedrig, so entstehen bei der Vernetzung Filme, die noch in organischen Lösemitteln wie Methyläthylketon löslich sind. Ist die Hydroxylzahl dagegen zu hoch, so wird der Film zu spröde und bleibt eventuell auch zu hydrophil. Im Molekül müssen mindestens zwei vernetzungsfähige, bevorzugt primäre Hydroxylgruppen vorliegen.

Die für den Vernetzungsprozeß wichtigen primären und/oder sekundären Hydroxylgruppen können anteilweise durch primäre und/oder sekundäre Aminogruppen ersetzt werden. Das Einführen von primären Aminogruppen in den Harzgrundkörper erfolgt vorzugsweise durch Umsetzen von mindestens ein, bevorzugt mindestens zwei Epoxidgruppen pro Molekül enthaltenden Harzen mit einem amino- und/oder hydroxylgruppenhaltigen Ketimin und/oder Aldimin und/oder Polyamin. Die Ketimine werden nach bekannten Methoden durch Wasserabspaltung aus den entsprechenden Polyaminen der allgemeinen Struktur R-NR-R-NH₂ oder den entsprechenden Aminoalkoholen der allgemeinen Struktur HO-R-NH₂ und den geeigneten aliphatischen Ketonen wie Diäthylketon, Methylisobutylketon, Äthyl-n-propylketon oder auch Cyclopentanon, Cyclohexanon, Acetophenon usw. hergestellt. Bevorzugte Ketimine sind Umsetzungsprodukte aus Methylisobutylketon und Diäthylentriamin. Die Reaktionsbedingungen (Reaktionstemperatur, Auswahl des Lösemittels) müssen so gewählt werden, daß keine die Ketiminbindung zersetzende Substanzen wie Wasser in dem Reaktionsprodukt vorhanden bleiben.

Das Ketimin schützt die primäre Aminogruppe (vgl. US-A 3,523,925), so daß das Amin über eine weitere funktionelle Gruppe, z.B. eine Hydroxylgruppe oder bevorzugt eine sekundäre Aminogruppe ohne Schwierigkeiten mit dem Epoxidgrundharz umgesetzt weden kann. Durch die Wahl der Molverhältnisse der eingesetzten Komponenten muß gewährleistet sein, daß kein unumgesetztes niedrigmolekulares Amin im Ansatz zurückbleibt, weil sonst durchbruchartige Oberflächenstörungen beim elektrophoretischen Beschichten auftreten. Die Reaktion der sekundären Aminogruppen des Polyaminoketimins mit der Epoxidgruppe beginnt schon bei Raumtemperatur und ist im allgemeinen exotherm. Um eine vollständige Umsetzung zu erreichen, ist es in der Regel notwendig, die Temperatur zeitweise auf 50 bis 120 °C zu erhöhen.

Die verkappten Polyisocyanate (Komponente B) werden dadurch hergestellt, daß man ein multifunktionelles Isocyanat mit mindestens einer stöchiometrischen Menge einer monofunktionellen, aktiven Wasserstoff (Zerewitinow--Reaktion) enthaltenden Verbindung umsetzt, wobei gegebenenfalls basische Katalysatoren wie tertiäre Amine oder geringe Mengen an Zinnsalzen wie Dibutylzinndilaurat zugegeben werden können. Die Isocyanatgruppe wird auf diese Weise bei Raumtemperatur gegen Reaktionen mit Wasser oder Alkoholen geschützt. Die Schutzgruppe spaltet sich bei Einbrenntemperaturen von weniger als 210 °C, vorzugsweise weniger als 190 °C, besonders unter 180 °C, andererseits über 110 °C, vorzugsweise über 150 °C, wieder ab, damit die freiwerdende Isocyanatgruppe mit den Hydroxylgruppen des Basisharzes reagieren kann. Mittel, die die Isocyanate blockieren, enthalten nur eine einzige Amin-, Amid-, Lactam-, Thio- oder Hydroxylgruppe. So haben sich beispielsweise bewährt aliphatische oder cycloaliphatische Alkohole wie 2-Äthylhexanol, Dialkylaminoalkohol wie Dimethylaminoäthanol, Oxime wie Methyläthylketoxime, Lactame wie ε-Caprolactam oder Pyrrolidon-2, Imide wie Phthalimid oder N-Hydroxy-maleinimid, Hydroxyalkylester, Malonsäureoder Acetessigsäureester. Es werden aber auch β-Hydroxyglykole oder -glykoläther und Glykolamide empfohlen. Bevorzugt sind auch solche Isocyanatverbindungen, bei denen ein Teil des Verkappungsmittels (vorzugsweise 10 bis 50 % mindestens eine olefinisch ungesättigte Gruppe enthält. Beispiele für entsprechende Verkappungsmittel sind die Hydroxyalkyl(meth)acrylate.

Als typische multifunktionelle Isocyanate eignen sich aliphatische, cycloaliphatische, araliphatische und/oder aromatische Polyisocyanate mit mindestens zwei Isocyanatgruppen pro Molekül. Als aromatische Diisocyanate und Polyisocyanate eignen sich die Isomeren oder Isomerengemische von Phenylendiisocyanat, Toluylendiisocyanat, Xylylendiisocyanat, Biphenylendiisocyanat, Naphthylendiisocyanat und Diphenylmethandiisocyanat, Diphenyltetraisocyanat, bevorzugt Naphthyltetraisocyanat, Toluylen-, Isophoron- und Xylylendiisocyanat. Aufgrund ihrer guten Beständigkeit gegenüber ultraviolettem Licht ergeben (cyclo)aliphatische Diisocyanate Produkte mit geringer Vergilbungsneigung. Beispiele hierfür sind Isophorondiisocyanat, Cyclopentylendiisocyanat, sowie die Hydrierungsprodukte der aromatischen Diisocyanate wie Cyclohexylendiisocyanat, Methylcyclohexylendiisocyanat und Dicyclohexylmethan-diisocyanat. Aliphatische Diisocyanate sind Verbindungen der Formel

O = C = N - (CR²)ᵣ - N = C = O

worin r eine ganze Zahl von 2 bis 20, insbesondere 6 bis 8 ist und R² Wasserstoff oder einen niedrigen Alkylrest mit 1 bis 8 C-Atomen, vorzugsweise 1 oder 2 C-Atomen bedeuten. Beispiele hierfür sind Trimethylendiisocyanat, Tetramethylendiisocyanat,Pentamethylendiisocyanat,Hexamethylendiisocyanat, Propylendiisocyanat, Äthyläthylendiisocyanat, Dimethyläthylendiisocyanat, Methyltrimethylendiisocyanat und Trimethylhexandiisocyanat. Besonders bevorzugt werden als Diisocyanate Isophorondiisocyanat, Dicyclohexylmethan-diisocyanat, Diphenylmethandiisocyanat, Trimethylendiisocyanat, Tetramethylendiisocyanat oder Toluylendiisocyanat.

Vinylpolymerisate, die Isocyanatgruppen enthalten und durch Copolymerisation von z. B. Cyanatoäthyl(meth)acrylat oder Dimethyl-isopropylbenzylisocyanat mit noch Alkyl(meth)acrylaten und/oder (Alkyl)-vinylbenzolen entstehen, können auch verwendet werden. Ebenso sind gemischte aliphatische/aromatische Isocyanat-Verbindungen geeignet.

Als Polyisocyanate haben sich auch Produkte bewährt, die durch Trimerisation oder Oligomerisation der oben genannten Diisocyanate oder durch Reaktion dieser Diisocyanate mit polyfunktionellen OH- oder NH-Gruppen enthaltenden Verbindungen entstehen. Falls notwendig, kann die mittlere Funktionalität gegebenenfalls durch Zusatz von Monoisocyanaten gesenkt werden. Beispiele für solche kettenabbrechenden Monoisocyanate sind Phenylisocyanat, Cyclohexylisocyanat und Stearylisocyanat.

Eine Molekülvergrößerung kann auch durch Reaktion mit tertiären Aminogruppen enthaltenden Polyalkoholen wie N-Methyl-diäthanolamin, Triäthanolamin oder tertiäre Aminogruppen enthaltenden Polyaminen wie 3-(Methyl)-3-(2-aminoäthyl)aminopropylen erfolgen. Um die Löslichkeit zu verbessern, können außerdem kettenabbrechende N-Dialkyl-aminoalkohole wie Dimethylaminoäthanol oder N,N-Dialkyl-alkylendiamine wie Dimethylaminopropylen oder N,N-Diäthyl-N'-methyl-1,2-äthandiamin eingesetzt werden. Isocyanathaltige Präpolymere auf Basis von Polyglykoläthern, Polyesterpolyolen, Polyätherpolyolen, Polycaprolactonpolyolen und/oder Polycaprolactampolyolen können ebenfalls mit Vorteil eingesetzt werden.

Zur Synthese des verkappten Polyisocyanats kann das entsprechende Monooder Di-isocyanat vor Zugabe des Verkappungsmittels in den zur Herstellung des Polymers C) benötigten olefinisch ungesättigten Verbindungen C) oder deren Mischungen oder in aprotischen Lösemitteln wie z.B. Toluol, Xylol oder Methyisobutylketon gelöst werden. Alternativ hierzu kann das ungesättigte Monomere bzw. aprotische Lösemittel wie die oben genannten auch nach der Addition des Verkappungsmittels zu dem Isocyanat zugegeben werden.

Eine andere Variante besteht darin, daß die beschriebenen Diisocyanate in stöchiometrisch geeigneter Weise mit den beschriebenen Monoalkoholen und/oder Aminen nur halbblockiert werden, wobei Umsetzungsprodukte von Butylglykol bzw. Butyldiglykol mit Toluylendiisocyanat und Methyläthylketoxim mit Isophoron- und Tetramethylxylylendiisocyanat bevorzugt sind. Die Halbverkappung kann lösemittelfrei in Substanz oder in den beschriebenen Monomeren durchgeführt werden. Solche halbblockierten Diisocyanate werden dann über die verbleibende NCO-Funktion entweder vor oder vorzugsweise nach der Umsetzung der Epoxidharze mit den Aminen an die freien Hydroxyl- bzw. Aminogruppen des Epoxidharzes bzw. des Amino-Epoxidharzes addiert, wobei diese Addition vorzugsweise in Gegenwart der beschriebenen Monomere durchgeführt wird. Auf diese Weise erhält man sogenannte selbstvernetzende Systeme, die ebenfalls Gegenstand dieser Erfindung sind. Zur Ausbalancierung anwendungstechnischer Eigenschaften kann einem selbstvernetzenden System anteilsweise ein blockiertes Polyisocyanat B) zugemischt werden.

Als ungesättigte Monomere, die bereits bei der Synthese des Amino-Epoxid-harzes als auch bei der Synthese des verkappten Isocyanats vorhanden sein können bzw. nach deren Synthese zugegeben werden, kommen die folgenden Vinylmonomeren in Frage: Vinylaromaten, wie Styrol, Methylstyrole, Halogenstyrole; Vinyläther; Vinylester von aliphatischen Monocarbonsäuren mit 2 bis 18 Kohlenstoffatomen, wie Vinylacetat, Vinylpropionat, Vinylstearat; Ester von α,β-ungesättigten Säuren wie Alkyl(meth)acrylate, Alkylfumarate, Alkylmaleinate mit 1 bis 12 Kohlenstoffatomen in der Alkylgruppe; Monoester von α,β-ungesättigten Säuren mit mehrwertigen Alkoholen wie Hydroxyalkyl(meth)acrylate, Hydroxyalkylcrotonate mit 2 bis 12 Kohlenstoffatomen und einer bis drei freien Hydroxylgruppen in der Alkoholgruppe wie Hydroxyäthylund Hydroxypropyl-(meth)acrylat, Neopentylglykolmono(meth)acrylat, Trimethylolpropanmono(meth)acrylat und Pentaerythritmono(meth)acrylat sowie Mischungen der genannten Vinylmonomeren.

Vorzugsweise werden eingesetzt Acryl- oder Methacrylsäureester von 1 bis 18 Kohlenstoffatome enthaltenden Monoalkoholen, vorzugsweise n-Butylmethacrylat, Methylmethacrylat, Isobutylacrylat, 2-Äthylhexylacrylat, insbesondere Butylacrylat in Betracht. Weitere geeignete Monomere sind Styrol, Vinyltoluol, α-Methylstyrol oder höher substituierte Styrole, wie z.B. 2,4-Dimethylstyrol, sowie Vinylester von 2 bis 15 Kohlenstoffatome enthaltenden Monocarbonsäuren, wie z.B. Vinylacetat, Vinylpropionat, Vinylpivalat, Vinylversatat. Bevorzugt sind hier insbesondere Styrol, Vinyltoluol und α-Methylstyrol, Weiterhin können als ungesättigte Monomere Hydroxyalkylacrylate, bevorzugt Hydroxyäthyl(meth)acrylat oder Hydroxypropyl(meth)acrylat oder ungesättigte 1,2-Epoxidgruppen enthaltende Monomere wie Glycidyl(meth)acrylat genommen werden. Monomere dieser Art können wegen ihrer reaktiven Gruppen erst nach Abschluß der Synthese des Amino-Epoxidharzes bzw. des verkappten Isocyanats zugefügt werden. Der Massenanteil an ungesättigten Monomeren bzw. an Polymer C) beträgt ca. 1 bis 80, vorzugsweise 5 bis 30 %, bezogen auf die Gesamtmasse der Komponenten A), B) und C), jeweils als Feststoff gerechnet.

Das Mischungsverhältnis der Komponenten A) zu B) liegt bevorzugt zwischen 90 zu 10 und 60 : 40 % und wird empirisch aus den optimal erreichbaren anwendungstechnischen Eigenschaften bei der gegebenen Einbrenntemperatur bestimmt. Die Komponenten A) und B), bevorzugt deren Mischungen mit den ungesättigten Monomeren, können in dem beschriebenen Verhältnis entweder kalt gemischt werden oder Komponente B) wird der in situ gefertigten Komponente A) bei höherer Temperatur zugesetzt. Anschließend werden in der Lackverarbeitung gebräuchliche Additive und Säuren zugegeben.

Die Vernetzung der OH-gruppenhaltigen Komponente A) mit blockierten Polyisocyanaten B) kann gegebenenfalls durch Zusatz von 0,01 bis 2 %, speziell 0,5 bis 1 %, bezogen auf die Summe der Massen der Komponenten A) und B), stark basischer tertiärer Amine und/oder aktiver Metallverbindungen beschleunigt werden. Eine besondere, manchmal synergistische Wirkung wird erreicht, wenn das abgeschiedene OH-gruppenhaltige Harz in einem stark basischen Medium vorliegt und Metallsalze von Wismut, Blei, Cobalt, Eisen, Antimon und/oder Zinn-II- und -IV genommen werden. Besonders bevorzugt werden Katalysatoren wie Eisen-lll-acetylacetonat, Dibutylzinndilaurat, Tri-n-butyl-zinn-oxid, Dibutylzinn-dioctylmaleat, Zinn-octoat, Zinn-oleat, Tetrabutyltitanat und/oder Cobalt-2-äthylhexoat.

Zum Ausbalancieren der anwendungstechnischen Eigenschaften ist es zweckmäßig, daß das an der Kathode abscheidbare Harz außer dem Vernetzungsmittel noch zusätzlich eine Massenanteil bis zu 15 %, bevorzugt 1 bis 10 % hydroxy- und/oderamino- und/oder epoxyfunktioneller doppelbindungshaltiger Monomere enthält, die mit den blockierten Polyisocyanaten und dem Amino-epoxidharz reagieren können.

Durch Protonieren mit Säuren wird das kationische Bindemittelgemisch in an sich bekannter Weise wasserverdünnbar gemacht. Beispiele für Säuren sind Ameisensäure, Milchsäure, Essigsäure, Propionsäure, Zitronensäure, Malonsäure, Acrylsäure, Phosphorsäure oder Alkylphosphorsäure. Einbasige niedermolekulare organische Carbonsäuren werden bevorzugt. Es muß mindestens so viel Säure zugegeben werden, daß eine stabile Emulgierung des kationischen Basisharzes erfolgt. Ein Überschuß an Säure, d.h. ein Neutralisationsgrad über 100 %, ist zweckmäßig zu vermeiden. Der "MEQ-Wert" (spezifischer Säuregehalt im Festharz, üblicherweise gemessen in mmol/100 g) liegt im allgemeinen zwischen 20 und 80 mmol/100 g. Es wird ein möglichst niedriger spezifischer Säuregehalt angestrebt, um ein möglichst hohes Abscheidequivalent zu erhalten. Diese Mischung wird dann mit vorzugsweise 60 bis 95 °C warmem, entmineralisierten Wasser zu einer Emulsion weiterverarbeitet. In der entstandenen Emulsion werden durch Zugabe von Radikalbildnern anschließend die vorhandenen ungesättigten Monomere nach bekannten Emulsionspolymerisationstechniken polymerisiert und man erhält 30 bis 60%ige, vorzugsweise 30 bis 40%ige Dispersionen. Anwendung als Radikal-Initiatoren können sowohl alle bekannten Redoxsysteme als auch thermisch zerfallende Radikalbildner wie Azoverbindungen, Peroxide, Persäureester und Hydroperoxide finden. Bevorzugt werden Kombinationen mit Reduktionsmitteln wie tert.-Butylhydroperoxid/Ascorbinsäure. Die Molmassen der so erhaltenen polymeren Harze C) liegen, bestimmt nach der Gelpermeationschromatographie-Methode, zwischen 10.000 und 2.000.000, vorzugsweise zwischen 30.000 und 600.000 g/mol. Zur Feineinstellung geeigneter Molmassen können gegebenenfalls Regler eingesetzt werden, wie z.B. Alkohole, Polyäther, Mercaptane oder Unterphosphorige Säure. Die Polymerisationstemperaturen liegen im allgemeinen zwischen ca. 20 und 95 °C. Sie richten sich nach dem anzustrebenden Molekulargewichtsbereich und den verwendeten Polymerisationsinitiatoren und deren Wirkungsoptimum.

Die erfindungsgemäßen Dispersionen eignen sich ausgezeichnet als ionisch stabilisierte Bindemittel auf Wasserbasis, die zur Weiterverarbeitung zu Elektrotauchlacken besonders geeignet sind. Diese Elektrotauchlacke auf der Basis der erfindungsgemäßen Dispersionen können Bleisilikat als Korrosionsschutzpigment enthalten. Ein besonderer Vorteil liegt jedoch darin, daß die erfindungsgemäßen Dispersionen sich für bleifreie Elektrotauchlacke eignen.

In den nachfolgenden Beispielen bedeuten alle Angaben von Prozent oder Teilen Massengehalte, sofern nicht anders angegeben.

### Beispiele

### Herstellungsbeispiele

### I. Styrolisierung von Bisphenol A (BisA-S)

In einen mit Rückflußkühler und Rührer ausgerüsteten 3 Halskolben werden 289,45 g Styrol, 317,30 g Bisphenol A, 0,57 g Borsäure und 1,35 g Oxalsäure gegeben und unter Schutzgas (Stickstoff) auf 120 °C aufgeheizt. Anschließend hält man solange bei 120 °C, bis ein Festkörpergehalt (1 g, 160 °C, 0,5 h) von über 95 % erreicht ist. Danach läßt man die Schmelze in Pfannen ab und zerkleinert den auf Raumtemperatur abgekühlten Feststoff.

### II. Epoxyharze

### Grundansatz:

In einem 6 l 4-Halskolben mit Rückflußkühler und Rührer wird eine auf 120 °C erwärmte Mischung von ®Epikote 828 R¹ und Bisphenol A bzw. styrolisiertem Bisphenol A-S (Beispiel I) mit einer 20%igen Katalysatorlösung von 4-Dimethylaminopyridin in ®Texanol² versetzt. Anschließend heizt man auf 150 °C und hält dort solange, bis der in Tabelle 1 erwähnte EV-Wert (molare Masse dividiert durch die Anzahl der Epoxidgruppen pro Molekül) erreicht ist. Danach wird die Heizung ausgeschaltet und mit Methoxypropanol bzw. Styrol auf einen Festkörpermassengehalt von 70 % bzw. 80 % verdünnt.

**Tabelle 1**

| Epoxyharz | 2 | 3 | 4* | 5^{†} |
|---|---|---|---|---|
| Epikote 828 | 1986,0 g | 1986,0 g | 1986,0 g | 1986,0 g |
| Bisphenol A | 589,0 g | 589,0 g | | |
| BisA-S aus Bsp. I | | | 1126,6 g | 1126,6 g |
| Katalysatorlsg. | 2,6 g | 2,6 g | 3,1 g | 3,1 g |
| EV-Wert (g/mol) | 451 | 451 | 545 | 545 |
| Methoxypropanol | 1104,0 g | - | 1334,0 g | - |
| Styrol | - | 644,0 g | - | 778 |
| | | | | |
| Festkörpergehalt | 70 % | 80 % | 70 % | 80 % |

| | | | | |
|---|---|---|---|---|
| * Vergleich | | | | |
| † erfindungsgemäß | | | | |

¹ Bisphenol A-Diglycidyläther (Deutsche Shell Chemie GmbH)
² Mischester-Lösungsmittel (Eastman Chemical Co.)

### 3. Epoxyaminaddukte

Das in Beispiel II (Tabelle 1) hergestellte Epoxyharz wird in einen 2 1 3-Halskolben mit Rückflußkühler und Rührer gegeben und mit Methoxypropanol bzw. Styrol verdünnt. Anschließend wird die Lösung bei 40 bis 50 °C mit Diäthanolamin versetzt. Es wird solange bei angegebener Temperatur gehalten, bis der in Tabelle 2 niedergelegte EV-Wert erreicht ist. Danach setzt man Dimethylaminopropylamin und ein Addukt aus 1 mol Hexamethylendiamin (HMDA) und 2 mol ®Cardura E 10³ (E 10) zu, heizt auf 120 °C und hält dort solange, bis ein EV-Wert von über 10 000 g/mol und die entsprechende Aminzahl erreicht ist.

**Tabelle 2**

| **Ansatz** | **2-2** | **3-2** | **4-2** | **5-2** |
|---|---|---|---|---|
| Epoxyharz (Tab. 1) | 920,4 g | 805,4 g | 1112,3 g | 973,3 g |
| Methoxypropanol | 162,1 g | - | 176,8 g | - |
| Styrol | - | 187,6 g | - | 211,7 g |
| Diäthanolamin | 52,5 g | 52,5 g | 52,5 g | 52,5 g |
| Dimethylaminopropylamin | 19,4 g | 19,4 g | 19,4 g | 19,4 g |
| Addukt (E 10 + HMDA) | 97,6 g | 97,6 g | 97,6 g | 97,6 g |
| | | | | |
| Aminzahl (Lsg.) | 53,7 | 57,8 | 46,1 | 49,6 |
| Festkörpermassengehalt | 65 % | 70 % | 65 % | 70 % |

³ Glycidylester der Versatic-10-Säure (2,2-Dimethyloctansäure, Deutsche Shell Chemie GmbH)

### IV. Urethanhärter

In einem 2 l 4-Halskolben mit Rückflußkühler, Destillationsbrücke und Rührer werden bei Raumtemperatur 522 g Toluylendiisocyanat und 544 g Toluol vorgelegt. Anschließend dosiert man eine Mischung von 134 g Trimethylolpropan und 354 g Butylglykol so zu, daß die Temperatur nicht über 100 °C ansteigt. Man rührt solange bei 90 bis 100 °C nach, bis der NCO-Wert unter 0.1 % beträgt und setzt dann 136,44 g ®Texanol und 54,58 g 2-Äthylhexanol zu.

### V. Dispersionen

a) Vorgehensweise für Ansatz 2-3 und 4-3 (Tabelle 3):
   In einem Reaktionsgefäß mit Rührer, Rückflußkühler und Destillationsbrücke wird die 65 %ige Harzlösung 2-2 bzw. 4-2 geladen, auf 85 °C aufgeheizt und das Lösemittel im Vakuum abdestilliert. Sobald kein Destillat mehr übergeht setzt man den in Beispiel IV hergestellten Härter zu. Nachdem man ca. 15 Minuten bei 85 °C homogenisiert hat, neutralisiert man mit Ameisensäure und dispergiert mit vollentsalztem Wasser. Man erhält feinteilige (Teilchengröße unter 500 nm) Dispersionen 2-3 und 4-3 mit einem Feststoffmassengehalt von 30 %.
b) Vorgehensweise für Ansatz 3-3 und 5-3 (Tabelle 3):
   In einem Reaktionsgefäß mit Rührer und Rückflußkühler wird die 70 %ige Harzlösung 3-2 bzw. 5-2 geladen und auf 90 °C erhitzt. Anschließend setzt man Dodecylmercaptan, Hydroxyäthylmethacrylat und den in Beispiel IV hergestellten Härter zu (Tabelle 3). Nach ca. 15 Minuten Nachrühren bei 90 °C wird mit Ameisensäure neutralisiert und mit vollentsalztem Wasser dispergiert.

Man setzt nun die Hälfte der angegebenen Menge an @Trigonox A 80⁴ zu, homogenisiert ca. 15 Minuten und dosiert anschließend über 20 Minuten eine 10 %ige Lösung von Ascorbinsäure in deionisiertem Wasser bei 90 °C zu.
Nach einer Stunde Reaktion bei 90 °C setzt man die zweite Hälfte Trigonox A 80 zu und läßt noch 2 Stunden bei 90 °C polymerisieren.
Man erhält feinteilige (Teilchengröße unter 500 nm) Dispersionen 3-3 und 5-3 mit einem Feststoffmassengehalt von 30 %.

**Tabelle 3**

| Ansatz | 2-3* | 3-3* | 4-3* | 5-3^{†} |
|---|---|---|---|---|
| Harzlösung aus Tabelle 2 | 1252,0 g | 1162,5 g | 1458,6 g | 1354,5 g |
| Destillat | 438,2 g | - | 510,0 g | - |
| Härter aus Bsp. IV | 430,2 g | 614,4 g | 501,1 g | 716,0 g |
| Dodecylmercaptan | - | 15,4 g | - | 17,9 g |
| Ameisensäure 85 % | 18,9 g | 27,4 g | 22,0 g | 31,4 g |
| Hydroxyäthylmethacrylat | - | 10,8 g | - | 12,6 g |
| Trigonox A 80 | - | 7,8 g | - | 9,1 g |
| Ascorbinsäure | - | 6,1 g | - | 7,1 g |
| Wasser, deionisiert | 2612,3 g | 3778,6 g | 3043,5 g | 4403,1 g |
| Festkörpermassengehalt | 30 % | 30 % | 30 % | 30 % |

| | | | | |
|---|---|---|---|---|
| * Vergleich | | | | |
| † erfindungsgemäß | | | | |

⁴ Peroxid-Initiator der Akzo Nobel Chemie

### VI. Klarlacke

Die unter Punkt V hergestellten Kunstharzdispersionen werden durch Verdünnung mit deionisiertem Wasser in Klarlacke mit einen Festkörpergehalt von 15 % überführt und 2 Stunden gerührt. Anschließend werden bei 32 °C unter Anlegen von Spannung und einer Abscheidezeit von 150 Sekunden Bleche beschichtet. Die Bleche werden 20 Minuten bei 180 °C eingebrannt. Man erhält einen glatten, kraterfreien Film. Die Filme werden zur Bestimmung der Glasübergangstemperatur (mittels DSC = dynamische Differenzkalo- metrie; Perkin Elmer DSC) und der komplexen Viskosität vom Blech abgelöst. Die Ergebnisse der Messungen sind in Tabelle 4 aufgeführt.

**Tabelle 4**

| Klarlack | KL-1° | KL-2° | KL-3° | KL-4^{†} |
|---|---|---|---|---|
| Dispersion | 2-3 | 3-3 | 4-3 | 5-3 |
| Spannung | 300 V | 300 V | 300 V | 300 V |
| Schichtdicke | 10 µm | 23 µm | 24 µm | 33 µm |
| | | | | |
| Glasübergangstemperatur | 39,5 °C | 36,2 °C | 40,2 °C | 34,2 °C |
| Viskosität*150°C, 0.01 Hz | 246 Pas | 120 Pas | 92,9 Pas | 66,5 Pas |

| | | | | |
|---|---|---|---|---|
| * Viskosität: Platte-Kegel-Messung der komplexen Viskosität durch Oszillation mittelsschub-spannungs-gesteuertem Rheometer *(Bohlin CS*). | | | | |
| ° Vergleich | | | | |
| † erfindungsgemäß | | | | |

## Patentansprüche

1. Wäßrige Kunstharzdispersionen enthaltend A) ein ionisches Harz auf Basis von aralkylierten Epoxidharzen, B) ein verkapptes Polyisocyanat und C) ein Polymer aus mindestens einem radikalisch polymerisierbaren olefinisch ungesättigten Monomeren, wobei die zur Synthese der Epoxidharze eingesetzten Bisphenole zum Teil oder alle mit je mindestens einer Aralkylgruppe substituiert sind.

2. Wäßrige Kunstharzdispersionen gemäß Anspruch 1, dadurch gekennzeichnet, daß als Komponente A kationische Harze auf Basis von aralkylierten Epoxidharzen verwendet werden.

3. Wäßrige Kunstharzdispersionen gemäß Anspruch 1, dadurch gekennzeichnet, daß als Komponente A kationische Epoxid-Amin-Harze auf Basis von aralkylierten Epoxidharzen verwendet werden.

4. Wäßrige Kunstharzdispersionen gemäß Anspruch 1, dadurch gekennzeichnet, daß als Komponente A kationische Epoxid-Amin-Harze auf Basis von aralkylierten Epoxidharzen mit einer Aminzahl von 30 bis 150 mg KOH/g verwendet werden.

5. Wäßrige Kunstharzdispersionen gemäß Anspruch 1, dadurch gekennzeichnet, daß als Komponente A kationische Epoxid-Amin-Harze auf Basis von aralkylierten Epoxidharzen mit einer Hydroxylzahl von 50 bis 500 mg KOH/g verwendet werden.

6. Wäßrige Kunstharzdispersionen gemäß Anspruch 1, dadurch gekennzeichnet, daß als Komponente A kationische Epoxid-Amin-Harze auf Basis von aralkylierten Epoxidharzen mit einer zahlenmittleren Molmasse (Mn) von 250 bis 10000 g/mol verwendet werden.

7. Wäßrige Kunstharzdispersionen gemäß Anspruch 1, dadurch gekennzeichnet, daß als Komponente A kationische Epoxid-Amin-Harze auf Basis von styrolisierten Epoxidharzen verwendet werden.

8. Wäßrige Kunstharzdispersionen gemäß Anspruch 1, dadurch gekennzeichnet, daß in dem ionischen aralkylierten Epoxidharz A ein Teil des aralkylierten Polyglycidyläthers durch aliphatische Polyglycidyläther der Formel 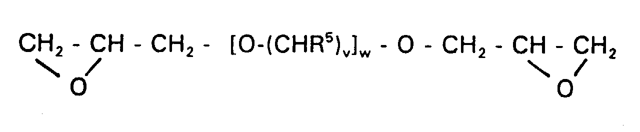 ersetzt wird, wobei
R⁵ H oder ein niedriger, gegebenenfalls mit verschiedenen Substituenten versehener Alkylrest ist und
v Zahlen von 2 bis 6 und
w Zahlen von 5 bis 50 bedeuten.

9. Wäßrige Kunstharzdispersionen gemäß Anspruch 1, dadurch gekennzeichnet, daß die zur Herstellung der kationischen Epoxid-Amin-Harze verwendeten Amine ausgewählt sind aus primären linearen und verzweigten Aminen wie Alkyl- und Alkanolaminen mit 1 bis 12 Kohlenstoffatomen im Alkyl- bzw. Alkanolrest, aus Dialkylaminoalkylaminen und Alkoxyalkylaminen mit insgesamt 3 bis 14 Kohlenstoffatomen, sekundären Aminen wie Dialkylaminen, Monoalkylhydroxyalkylaminen, Dihydroxyalkylaminen und cyclischen Aminen mit insgesamt 2 bis 14 Kohlenstoffatomen.

10. Wäßrige Kunstharzdispersionen gemäß Anspruch 1, dadurch gekennzeichnet, daß zur Herstellung der kationischen Epoxid-Amin-Harze auch primäre Amine der allgemeinen Formel
H₂N-CR¹R²-R³-O(CHR⁴-CHR⁵O)ₙR⁶
und/oder sekundäre Amine der allgemeinen Formel
H-N-CR¹R²-R³-O(CHR⁴-CHR⁵O)ₙ-R³-CR¹R²-N-H
eingesetzt werden, wobei
R¹ und R² für Wasserstoff, Alkyl-, oder -CH₂-OH Gruppen,
R³ für einen linearen oder verzweigten Alkylenrest, insbesondere für einen Alkvlenrest mit 1 bis 3 Kohlenstoffatomen.
R⁴ und R⁵ für Wasserstoff oder Alkylreste mit 1 bis 4 Kohlenstoffatomen,
R⁶ für Wasserstoff, einen Alkyl-, Cycloalkyl- oder Phenylrest, vorzugsweise für einen Alkylrest mit 1 bis 6 Kohlenstoffatomen und
n für eine ganze Zahl von 0 bis 5 stehen.

11. Wäßrige Kunstharzdispersionen gemäß Anspruch 1, dadurch gekennzeichnet, daß die Komponente B ausgewählt ist aus verkappten aliphatischen, araliphatischen, cycloaliphatischen und aromatischen Di- und Polyisocyanaten, Mischungen aus solchen Isocyanaten und Mischungen aus Di- und Polyisocyanaten mit Monoisocyanaten.

12. Wäßrige Kunstharzdispersionen gemäß Anspruch 1, dadurch gekennzeichnet, daß ein Teil der Verkappungsmittel der eingesetzten Isocyanatverbindungen mindestens eine olefinisch ungesättigte Gruppe enthält.

13. Wäßrige Kunstharzdispersionen gemäß Anspruch 1, dadurch gekennzeichnet, daß die Komponente C ausgewählt ist aus den Vinylmonomeren Vinylaromaten, wie Styrol, Methylstyrole, Halogenstyrole; Vinyläthern; Vinylestern von aliphatischen Monocarbonsäuren mit 2 bis 18 Kohlenstoffatomen, wie Vinylacetat, Vinylpropionat, Vinylstearat; Ester von α,β-ungesättigten Säuren wie Alkyl(meth)acrylate, Alkylfumarate, Alkylmaleinate mit 1 bis 12 Kohlenstoffatomen in der Alkylgruppe; Monoester von α,β-ungesättigten Säuren mit mehrwertigen Alkoholen wie Hydroxyalkyl(meth)acrylate, Hydroxyalkylcrotonate mit 2 bis 12 Kohlenstoffatomen und einer bis drei freien Hydroxylgruppen in der Alkoholgruppe wie Hydroxyäthyl- und Hydroxypropyl-(meth)acrylat, Neopentylglykolmono(meth)acrylat, )acrylat, Trimethylolpropanmono(meth)acrylat und Pentaerythritmono(meth)acrylat )acrylat sowie aus Mischungen der genannten Vinylmonomeren.

14. Verfahren zur Herstellung der wäßrigen Kunstharzdispersionen nach Anspruch 1, dadurch gekennzeichnet, daß eine Mischung aus mindestens einem Vinylmonomer C, einem ionischen Epoxidharz A und einem teilweise oder vollständig blockierten Di- oder Polyisocyanat in eine wäßrige Dispersion überführt wird und unter Zusatz eines radikalischen Initiators polymerisiert wird.

15. Verwendung der wäßrigen Dispersionen gemäß Anspruch 1 als Bindemittel in Elektrotauchlackbädern.

16. Verwendung der wäßrigen Dispersionen gemäß Anspruch 1 als Bindemittel in bleifreien kationischen Elektrotauchlackbädern.

17. Gegenstände, die mit eine Kunstharzdispersion gemäß Anspruch 1 beschichtet sind.

## Claims

1. An aqueous synthetic resin dispersion comprising A) an ionic resin based on aralkylated epoxy resins, B) a blocked polyisocyanate and C) a polymer of at least one olefinically unsaturated monomer which is capable of undergoing free-radical polymerization, some or all of the bisphenols employed for the synthesis of the epoxy resins being substituted with in each case at least one aralkyl group.

2. An aqueous synthetic resin dispersion as claimed in claim 1, wherein cationic resins based on aralkylated epoxy resins are used as component A.

3. An aqueous synthetic resin dispersion as claimed in claim 1, wherein cationic epoxy-amine resins based on aralkylated epoxy resins are used as component A.

4. An aqueous synthetic resin dispersion as claimed in claim 1, wherein cationic epoxy-amine resins based on aralkylated epoxy resins having an amine number of from 30 to 150 mg of KOH/g are used as component A.

5. An aqueous synthetic resin dispersion as claimed in claim 1, wherein cationic epoxy-amine resins based on aralkylated epoxy resins having a hydroxyl number of from 50 to 500 mg of KOH/g are used as component A.

6. An aqueous synthetic resin dispersion as claimed in claim 1, wherein cationic epoxy-amine resins based on aralkylated epoxy resins having a number-average molecular mass (Mn) of from 250 to 10,000 g/mol are used as component A.

7. An aqueous synthetic resin dispersion as claimed in claim 1, wherein cationic epoxy-amine resins based on styrenized epoxy resins are used as component A.

8. An aqueous synthetic resin dispersion as claimed in claim 1, wherein, in the ionic aralkylated epoxy resin A, some of the aralkylated polyglycidyl ether is replaced by aliphatic polyglycidyl ethers of the formula 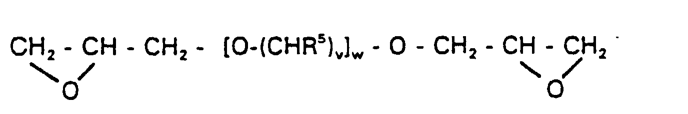 where
R⁵ is H or a lower alkyl radical which is provided if desired with various substituents, and
v is a number from 2 to 6, and
w is a number from 5 to 50.

9. An aqueous synthetic resin dispersion as claimed in claim 1, wherein the amines used for the preparation of the cationic epoxy-amine resins are selected from primary linear and branched amines such as alkylamines and alkanolamines having 1 to 12 carbon atoms in the alkyl or alkanol radical, from dialkylaminoalkylamines and alkoxyalkylamines having a total of 3 to 14 carbon atoms, secondary amines such as dialkylamines, monoalkylhydroxyalkylamines, dihydroxyalkylamines and cyclic amines having a total of 2 to 14 carbon atoms.

10. An aqueous synthetic resin dispersion as claimed in claim 1, wherein the amines employed for the preparation of the cationic epoxy-amine resins include primary amines of the formula
H₂N-CR¹R²-R³-O(CHR⁴-CHR⁵O)ₙR⁶
and/or secondary amines of the formula
H-N-CR¹R²-R³-O(CHR⁴-CHR⁵O)ₙ-R³-CR¹R²-N-H
where
R¹ and R² are hydrogen, or alkyl or -CH₂-OH groups,
R³ is a linear or branched alkylene radical, especially an alkylene radical having 1 to 3 carbon atoms,
R⁴ and R⁵ are hydrogen or alkyl radicals having 1 to 4 carbon atoms,
R⁶ is hydrogen, an alkyl, cycloalkyl or phenyl radical, preferably an alkyl radical having 1 to 6 carbon atoms, and
n is an integer from 0 to 5.

11. An aqueous synthetic resin dispersion as claimed in claim 1, wherein component B is selected from blocked aliphatic, araliphatic, cycloaliphatic and aromatic di- and polyisocyanates, mixtures of such isocyanates and mixtures of di- and polyisocyanates with monoisocyanates.

12. An aqueous synthetic resin dispersion as claimed in claim 1, wherein a section of the blocking agents of the isocyanate compounds employed contains at least one olefinically unsaturated group.

13. An aqueous synthetic resin dispersion as claimed in claim 1, wherein component C is selected from the vinyl monomers comprising aromatic vinyl compounds, such as styrene, methylstyrenes and halostyrenes; vinyl ethers; vinyl esters of aliphatic monocarboxylic acids having 2 to 18 carbon atoms, such as vinyl acetate, vinyl propionate and vinyl stearate; esters of α,β-unsaturated acids such as alkyl (meth)acrylates, alkyl fumarates and alkyl maleates having 1 to 12 carbon atoms in the alkyl group; monoesters of α,β-unsaturated acids with polyhydric alcohols, such as hydroxyalkyl (meth)acrylates, hydroxyalkyl crotonates having 2 to 12 carbon atoms and from one to three free hydroxyl groups in the alcohol group, such as hydroxyethyl and hydroxypropyl (meth)acrylate, neopentylglycol mono(meth)acrylate, trimethylolpropane mono(meth)acrylate and pentaerythritol mono(meth)acrylate, and from mixtures of the vinyl monomers mentioned.

14. A process for the preparation of an aqueous synthetic resin dispersion as claimed in claim 1, which comprises converting a mixture of at least one vinyl monomer C, one ionic epoxy resin A and one partially or completely blocked di- or polyisocyanate into an aqueous dispersion and polymerizing this dispersion with the addition of a free-radical initiator.

15. The use of an aqueous dispersion as claimed in claim 1 as a binder in electrodeposition coating baths.

16. The use of an aqueous dispersion as claimed in claim 1 as a binder in lead-free cationic electrodeposition coating baths.

17. An article coated with a synthetic resin dispersion as claimed in claim 1.

## Revendications

1. Dispersions aqueuses de résine synthétique contenant A) une résine ionique à base de résines époxydes aralkylées, B) un polyisocyanate coiffé et C) un polymère d'au moins un monomère à insaturation oléfinique polymérisable par voie radicalaire, les bisphénols utilisés pour la synthèse des résines époxydes étant substitués en partie ou tous par au moins un groupe aralkyle.

2. Dispersions aqueuses selon la revendication 1, caractérisées en ce que l'on emploie, en tant que composant A, des résines cationiques à base de résines époxydes aralkylées.

3. Dispersions aqueuses selon la revendication 1, caractérisées en ce que l'on emploie, en tant que composant A, des résines époxydes cationiques à fonction amino à base de résines époxydes aralkylées.

4. Dispersions aqueuses selon la revendication 1, caractérisées en ce que l'on emploie, en tant que composant A, des résines époxydes cationiques à fonction amino à base de résines époxydes aralkylées, ayant un indice d'amine de 30 à 150 mg KOH/g.

5. Dispersions aqueuses selon la revendication 1, caractérisées en ce que l'on emploie, en tant que composant A, des résines époxydes cationiques à fonction amino à base de résines époxydes aralkylées, ayant un indice d'hydroxyle de 50 à 500 mg KOH/g.

6. Dispersions aqueuses selon la revendication 1, caractérisées en ce que l'on emploie, en tant que composant A, des résines époxyde cationiques à fonction amino à base de résines époxydes aralkylées, ayant une masse molaire moyenne en nombre (Mn) de 250 à 10000 g/mole.

7. Dispersions aqueuses selon la revendication 1, caractérisées en ce que l'on emploie, en tant que composant A, des résines époxydes cationiques à fonction amino à base de résines époxydes styrénées.

8. Dispersions aqueuses selon la revendication 1, caractérisées en ce que, dans la résine ionique époxyde aralkylée, une partie des éthers polyglycidyliques aralkylés est remplacée par des éthers polyglycidyliques aliphatiques de formule 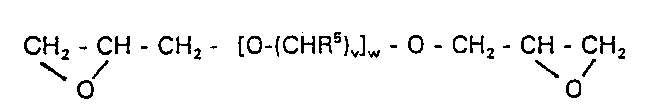 dans laquelle
R⁵ représente H ou un reste alkyle avec 1 à 6 atomes de carbone, éventuellement porteur de différents substituants et
v représente des nombres de 2 à 6 et
w représente des nombres de 5 à 50.

9. Dispersions aqueuses selon la revendication 1, caractérisées en ce que les amines nécessaires pour préparer les résines époxydes cationiques à fonction amino sont choisies parmi les amines primaires linéaires et ramifiées, comme des alkyl- et alcanolamines avec 1 à 12 atomes de carbone dans le reste alkyle, respectivement alcanol, des dialkylaminoalkylamines et des alkoxyalkylamines avec au total de 3 à 14 atomes de carbone, des amines secondaires, comme des dialkylamines, des monohydroxyalkylamines, des dihydroxyalkylamines et des amines cycliques avec au total de 2 à 14 atomes de carbone.

10. Dispersions aqueuses selon la revendication 1, caractérisées en ce que l'on emploie pour la préparation des résines époxydes cationiques à fonction amino des amines primaires de formule générale
H₂N-CR¹R²-R³-O(CHR⁴-CHR⁵O)ₙR⁶
et/ou des amines secondaires de formule générale
H-N-CR¹R²-R³-O(CHR⁴-CHR⁵O)ₙ₋R³-CR¹R²-N-H,
dans lesquelles
R¹ et R² représentent un atome d'hydrogène, des groupes alkyle ou -CH₂-OH,
R³ représente un reste alkylène linéaire ou ramifié, en particulier un reste alkylène avec 1 à 3 atomes de carbone,
R⁴ et R⁵ représentent un atome d'hydrogène ou des restes alkyle avec 1 à 4 atomes de carbone,
R⁶ représente un atome d'hydrogène, des restes alkyle, cycloalkyle ou phényle, de préférence un reste alkyle avec 1 à 6 atomes de carbone et
n est un entier de 0 à 5.

11. Dispersions aqueuses selon la revendication 1, caractérisées en ce que le composant B est choisi parmi les di- et polyisocyanates coiffés aliphatiques, cycloaliphatiques et aromatiques, des mélanges de tels isocyanates et des mélanges de di- et de polyisocyanates avec des monoisocyanates.

12. Dispersions aqueuses selon la revendication 1, caractérisées en ce qu'une partie de l'agent de coiffage des composés d'isocyanate employés contient au moins un groupe à insaturation oléfinique.

13. Dispersions aqueuses selon la revendication 1, caractérisées en ce que le composant C est choisi parmi les monomères vinyliques, les composés vinylaromatiques, comme le styrène, les méthylstyrènes, les halogénostyrènes ; les éthers vinyliques ; les esters vinyliques des acides monocarboxyliques aliphatiques avec 2 à 18 atomes de carbone, comme l'acétate de vinyle, le propionate de vinyle, le stéarate de vinyle ; les esters d'acides insaturés en α,β, comme des (méth)acrylates d'alkyles, des fumarates d'alkyles, des maléates d'alkyles avec 1 à 12 atomes de carbone dans le groupe alkyle ; les monoesters d'acides insaturés en α,β avec des alcools multifonctionnels, comme des (méth)acrylates d'hydroxyalkyles, des crotonates d'hydroxyalkyles avec 2 à 12 atomes de carbone et un à trois groupes hydroxyle libres dans le groupe alcool, comme le (méth)acrylate d'hydroxyéthyle et d'hydroxypropyle, le mono(méth)acrylate de néopentylglycol, le mono(méth)acrylate de triméthylol propane et le mono(méth)acrylate de pentaérythritol, ainsi que des mélanges des monomères vinyliques précités.

14. Procédé de préparation de dispersions aqueuses de résines synthétiques selon la revendication 1, caractérisé en ce que l'on transforme un mélange d'au moins un monomère vinylique C, d'une résine époxyde ionique A et d'un di- ou polyisocyanate partiellement ou entièrement bloqué en une dispersion aqueuse et on polymérise par ajout d'un amorceur de polymérisation radicalaire.

15. Utilisation des dispersions aqueuses selon la revendication 1 en tant que liant dans des vernis de trempage par électrodéposition.

16. Utilisation des dispersions aqueuses selon la revendication 1 en tant que liants dans des vernis de trempage par électrodéposition cationiques sans plomb.

17. Objets revêtus d'une dispersion de résine synthétique selon la revendication 1.
